(19) Europäisches Patentamt
European Patent Office
Office européen des brevets

(11) **EP 4 458 251 A1**

(12) **EUROPEAN PATENT APPLICATION**

(43) Date of publication:
**06.11.2024   Bulletin 2024/45**

(51) International Patent Classification (IPC):
***A61B 3/10*** *(2006.01)*      ***A61B 3/12*** *(2006.01)*
***A61B 3/13*** *(2006.01)*

(21) Application number: **23171187.0**

(22) Date of filing: **02.05.2023**

(52) Cooperative Patent Classification (CPC):
**A61B 3/13; A61B 3/10; A61B 3/12**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Sharpeye**
**94250 Gentilly (FR)**

(72) Inventors:
• **MAZLIN, Viacheslav**
  **75013 PARIS (FR)**
• **ALHADDAD, Samer**
  **75014 PARIS (FR)**
• **BOCCARA, Albert Claude**
  **75006 PARIS (FR)**

(74) Representative: **Icosa**
  **83 avenue Denfert-Rochereau**
  **75014 Paris (FR)**

(54)   **SYSTEM AND METHOD FOR IN VIVO CELLULAR RESOLUTION TRANSMISSION INTERFERENCE IMAGING OF AN EYE**

(57)   The present invention relates to a system (1) for in vivo, full-field transmission interference cellular resolution imaging of an eye (6) of an individual and to a method (100) for in vivo, full-field transmission interference imaging of an eye (6) of an individual.

**FIG. 1A**

EP 4 458 251 A1

**Description**

**FIELD OF INVENTION**

The present invention relates to in vivo eye imaging.

More precisely, the invention pertains to a system for in vivo cellular resolution imaging of an eye of an individual and to a method for in vivo cellular resolution of an eye of an individual.

**BACKGROUND OF INVENTION**

Optical imaging plays the key role in everyday diagnosis of ocular health. Today every ophthalmological hospital is equipped with the slit-lamp biomicroscope, fundus camera and optical coherence tomography (OCT) devices that provide a global view of the eye. Although these technologies can address the majority of ocular conditions, a number of important diseases remain underdiagnosed, for example dry eye condition (340 million people affected), glaucoma (80 million affected), etc. In order to be effectively treated these diseases require early and accurate diagnosis, which is difficult to achieve having only the global view of the eye lacking the resolution. In principle earlier and more accurate diagnosis as well as a better disease understanding can be achieved with the higher resolution diagnostic instrument that could evaluate the ocular health on the cellular level. Unfortunately, higher resolution imaging is not widely available in today's clinics mostly due to the limitations of the current technologies.

There have been several attempts to create a high-resolution imager for clinics. Confocal microscopy imaging method provides cell resolution of in vivo anterior eye useful for diagnosis of several diseases, such as dry eye or endothelial dystrophies. However, this technique requires physical contact with the eye. This requirement pushed back its adoption in clinics and reduced the number of imaged patients. Another class of imaging instruments based on adaptive optics achieved cellular resolution in the retina, however these instruments are complex to align and costly, currently accessible to only a few large clinical centers.

The invention aims at proposing a solution to the situation.

**SUMMARY**

This invention thus relates to a system for in vivo, full-field transmission interference cellular resolution imaging of an eye of an individual, the eye having an outer surface, the system comprising:

- an object arm intended to receive, when the system is in operation, the eye of the individual in an imaging plane, said object arm having an axis coincident with an imaging axis,
- an illumination arm comprising a light source coupled to a first optical lens and to a polarization beam splitter, configured to produce together an illumination beam, wherein the illumination beam is substantially collimated and symmetric with respect to the axis of the object arm,

   wherein, in operation, at least part of the illumination beam penetrates the eye of the individual, focuses onto an area of an internal reflective layer of the eye, and is backreflected by said internal reflective layer to form incident light waves,
   wherein, in operation, the imaging plane is located between the internal reflective layer of the eye and the outer surface of the eye,

- a detection arm comprising a tube lens and a bidimensional acquisition device, the bidimensional acquisition device comprising a plurality of sensors arranged in a detection plane, configured to acquire at least one bidimensional interferometric signal resulting from interferences between a substantially collimated reference wave obtained by transmission of said incident light waves and a divergent sample wave obtained by scattering said incident waves by a voxel of a slice of the eye of the individual, the detection plane being optically conjugated with said imaging plane by an optical system comprising said polarization beam splitter,

   wherein the divergent sample wave presents an additional phase shift relatively to the substantially collimated reference wave,
   wherein, the polarization beam splitter is configured to polarize the illumination beam and to filter out, in operation, light specularly reflected by the eye of the individual.

This system presents the advantages to be easy to align due to its symmetry. Also, the system is more robust and less sensitive to unwanted ocular motion given the common path between the object arm and the illumination arm.

In addition, the system allows to obtain high contrast image thanks to the filtering of stray light with the polarization beam splitter. This system is working in transmission, which allows eliminating reflection artefacts.

Advantageously, the illumination beam is of spatially incoherent light. Using a spatially incoherent source allows to avoid speckle in the images.

Advantageously, the internal reflective layer of the eye is the sclera of the eye of the individual.

Preferably, the illumination beam presents a divergence angle of less than 20 degrees, the divergence angle being measured with respect to the axis of the object arm, and wherein one dimension of said area of the internal reflective layer of the eye, when illuminated by the illumination beam is less than 6 millimeters. With this limited divergence angle, the illumination beam is substantially collimated which allows obtaining high contrast images.

Advantageously, the light source is positioned in a source plane, the system further comprising an aperture positioned in the source plane, and the aperture being configured to control the divergence angle of the illumination beam. The aperture allows controlling the collimation degree of the illumination beam, and thus the contrast of the images obtained with the system.

Advantageously, the system further comprises a linear polarizer positioned between the polarization beam splitter and the light source or between the polarization beam splitter and the bidimensional acquisition device. Using linear polarizers helps improving the polarization degree of the different beams travelling in the system and thus improving the interferometric contrast.

In some embodiments, the system further comprises a processing unit and a tunable lens positioned between the polarization beam splitter and the bidimensional acquisition device, the tunable lens having a controllable focal length and being configured to be controlled so as to acquire a plurality of bidimensional interferometric signals to determine a plurality of working images, each among the plurality of working images corresponding to a distinct imaging plane along the imaging axis, wherein the processing unit is configured to compute a 3D image of the eye of the individual based on the plurality of working images. Having a controllable element such as a tunable lens allows varying the position of the imaging plane, therefore acquiring several images depth wise along the imaging axis and thus along the depth of the eye of the individual under examination.

In some embodiments, the controlling of said tunable lens focal length, so as acquire a plurality of bidimensional interferometric signals depth wise inside a section of the eye of the individual, results in a plurality of predetermined phase differences between the reference wave and the sample wave comprised between $-\pi/2$ and $+\pi/2$, and wherein the processing unit is further configured to compute a sectioning image, based on a linear combination of said bidimensional interferometric signals among the plurality of bidimensional interferometric signals.

Advantageously, the system further comprises an optical multiplexing system positioned between the polarization beam splitter and the bidimensional acquisition device, so as to acquire in a single acquisition a plurality of bidimensional interferometric signals depth wise inside a section of the eye of the individual, the optical multiplexing system being configured to provide predetermined phase differences between the reference wave and the sample wave comprised between $-\pi/2$ and $+\pi/2$.

In some embodiments:

- the system further comprises a second optical lens positioned in the illumination arm or in the object arm,
- the object arm further comprises a third optical lens,
- the second optical lens is configured to produce a focused beam from the illumination beam so that the focused beam focuses in a back focal plane of said third lens, so that a substantially collimated beam comes out of the third optical lens.

In those embodiments, the system allows obtaining images of the anterior part of the eye (cornea, crystalline lens) under examination.

Preferably, the third optical lens is a microscope objective, a turret of microscope objectives or a zoom microscope objective, those objectives having a numerical aperture of 0.25 or higher.

Advantageously, the system further comprises an optical mask placed in a plane referred to as mask plane between the polarization beam splitter and the bidimensional acquisition device, and a lens or a system of lenses conjugating the back focal plane of the third lens with the mask plane, the optical mask being configured to partially block part of the reference wave, so as to improve image contrast. By optical mask, it is meant an element that selectively blocks fraction of the illumination incident on it. Typically, the optical mask is either the transparent glass plate partially covered with partially opaque paint or material. The optical mask can be also be made entirely without glass (see https://microcosmos.store/products/oblique-illumination-and-dark-field-filters). Typically, the partially opaque part is located in the center of the light beam in order to reduce the reference light. By selecting the thickness and type of the mask material we can control the transparency of the mask and the degree of the reference light suppression.

In some embodiments, the system further comprises a moving platform on which said third lens is mounted and a processing unit, the moving platform being configured to move along the imaging axis so as to acquire a plurality of bidimensional interferometric signals to determine a plurality of working images, each among the plurality of working images corresponding to a distinct imaging plane along the axis of the object arm, and wherein the processing unit is configured to, when moving said platform along said an imaging axis, compute a 3D image of the three-dimensional sample based on the plurality of working images. Having a moving element such as a moving platform allows for moving the imaging plane along the imaging axis, and thus acquiring several images depthwise along the imaging axis and within the depth of the eye of the individual under examination.

In some embodiments, moving the moving platform along said imaging axis, so as acquire the plurality of bidimensional interferometric signals depth wise inside a section of the eye of the individual, results in a plurality of predetermined phase differences between the reference wave and the sample wave comprised between $-\pi/2$ and $+\pi/2$, and the processing unit is further configured to compute a sectioning image, based on a linear combination of said bidimensional interferometric signals among the plurality of bidimensional interferometric signals.

In some embodiments, the system further comprises a moving platform on which the tube lens is mounted and a processing unit, the moving platform being configured to move along the imaging axis so as to acquire a plurality of bidimensional interferometric signals to determine a plurality of working images, each among the plurality of working images corresponding to a distinct imaging plane along the axis of the object arm, and wherein the processing unit is configured to, when moving said platform along said an imaging axis, compute a 3D image of the three-dimensional sample based on the plurality of working images. Having a moving element such as a moving platform allows for moving the imaging plane along the imaging axis, and thus acquiring several images depthwise along the imaging axis and within the depth of the eye of the individual under examination.

In some embodiments, moving the moving platform along said imaging axis, so as acquire the plurality of bidimensional interferometric signals depth wise inside a section of the eye of the individual, results in a plurality of predetermined phase differences between the reference wave and the sample wave comprised between $-\pi/2$ and $+\pi/2$, and the processing unit is further configured to compute a sectioning image, based on a linear combination of said bidimensional interferometric signals among the plurality of bidimensional interferometric signals.

In some embodiments, the system further comprises an additional eye imaging system, such as a fundus camera or a slit lamp system, an additional axial position tracking system such as an optical coherence tomography device, a macroview camera system, a stereo camera, a pupil camera, the additional eye imaging system or the additional axial position tracking system being integrated by means of a dichroic mirror placed in the illumination arm, in the object arm or between the beam splitter and the bidimensional acquisition device.

Another aspect of the invention pertains to a method for in vivo, full-field transmission interference imaging of an eye of an individual, the method comprising:

a) disposing the eye at an imaging plane in an object arm of a system for in vivo, full-field transmission interference imaging,
b) illuminating with a substantially collimated, spatially incoherent and symmetric illumination beam the eye of the individual so that the illumination beam focuses on a substantially small area of an internal reflective layer of the eye and is back reflected by said internal reflective layer to form incident light waves,
c) acquiring, by a dimensional acquisition device comprising a plurality of sensors arranged in a detection plane, at least one bidimensional interferometric signal,

the at least one bidimensional interferometric signal resulting from interferences between a reference wave obtained by transmission of said incident light waves, and a sample wave obtained by scattering said incident light waves by a voxel of a slice of the eye of the individual,
the imaging plane being conjugated with said detection plane,

d) obtaining at least one first image from the at least one bidimensional interferometric signal by means of a processing unit of said system.

In some embodiments, at step d), a plurality of first images are obtained at distinct timepoints, and the method further comprises:

- computing an average image, the average image being a linear combination of said plurality of first images,
- obtaining an effective first image at a timepoint subsequent to said distinct timepoints, with the method for in vivo, full-field transmission interference imaging previously described,
- obtaining a contrast-improved image by subtracting the computed average image to the effective first image.

In some embodiments, the method further comprises:

- training an artificial neural network, based on at least one training set of images of a training sample, each training set including a first training image and a second training image of a same section of the training sample obtained by averaging different images obtained with the method for in vivo, full-field transmission interference imaging previously described, the training comprising:

  - for each training set, providing the corresponding first training image to said artificial neural network as an input,
  - providing the corresponding second training image as a target output of said artificial neural network,

the method further comprising obtaining a denoised image as an output of the trained artificial neural network obtained by inputting the obtained at least one image at step d) to the trained artificial neural network.

In some embodiments, the method further comprises segmenting and counting cells and nerves within the obtained at least one first image.

In some embodiments, when the system for in vivo, full-field transmission interference imaging further comprises an additional position tracking system, at step d), at least one en-face image is obtained with the system for in vivo, full-field transmission interference imaging at at least one timepoint, the method further comprising:

- for each among the at least one en-face image, acquiring at least one corresponding image with the additional position tracking system;
- computing, based on the at least one corresponding image acquired with the additional position tracking system an axial position of the eye of the individual along the imaging axis over time.

In some embodiments, at step c), a set of bidimensional interferometric signals is acquired, and the method further comprises computing, using the processing unit of the system for in vivo, full-field transmission interference imaging, an image referred to as dynamics image, said dynamics image being representative of temporal variations of intensity between said bidimensional interferometric signals among the set of bidimensional interferometric signals.

## BRIEF DESCRIPTION OF THE DRAWINGS

**Figures 1a and 1b** are schematic diagrams of a system for in vivo, full-field transmission interference imaging of an eye of an individual according to some embodiments referred to as retinal imaging embodiments.

**Figures 2a and 2b** are schematic diagrams of part of a system for in vivo, full-field transmission interference imaging of an eye of an individual according to some embodiments, where an illumination divergence angle of an illumination beam is illustrated, according to respectively a retinal imaging embodiment and a corneal imaging embodiment.

**Figures 3a and 3b** are schematic diagrams of a system for in vivo, full-field transmission interference imaging of an eye of an individual according to some embodiments referred to as corneal imaging embodiments.

**Figure** 4 is a schematic diagram of a system for in vivo, full-field transmission interference imaging of an eye of an individual according to another corneal imaging embodiment.

**Figures 5a and 5b** are schematic diagrams of examples of a system for in vivo, full-field transmission interference imaging of an eye of an individual according to some embodiments, where the system comprises a controllable element.

**Figures 6a and 6b** are schematic diagrams of part of a system for in vivo, full-field transmission interference imaging of an eye of an individual according to some embodiments, where the system comprises an optical multiplexing system.

**Figure** 7 is a schematic diagram of a system for in vivo, full-field transmission interference imaging of an eye of an individual according to some embodiments, where the system comprises an additional eye imaging system.

**Figures 8a and 8b** are schematic diagrams of a system for in vivo, full-field transmission interference imaging of an eye of an individual according to some embodiments, where the system allows for partial suppression of a reference wave.

**Figure** 9 is an example of a flowchart representing the main steps of a method for in vivo, full-field transmission interference imaging of an eye of an individual according to some embodiments.

**Figures 10a, 10b, 10c, 10d, 10e, 10f and 10g** are images of internal reflective layers of human eye, such as retinal images and corneal images.

**Figures 11a, 11b and 11c** are respectively two interferometric images of a corneal stroma and an optical section resulting from their difference.

**DETAILED DESCRIPTION**

This invention relates to a system 1 for in vivo cellular resolution imaging of an eye of an individual and to a method for in vivo cellular resolution of an eye of an individual using this system 1.

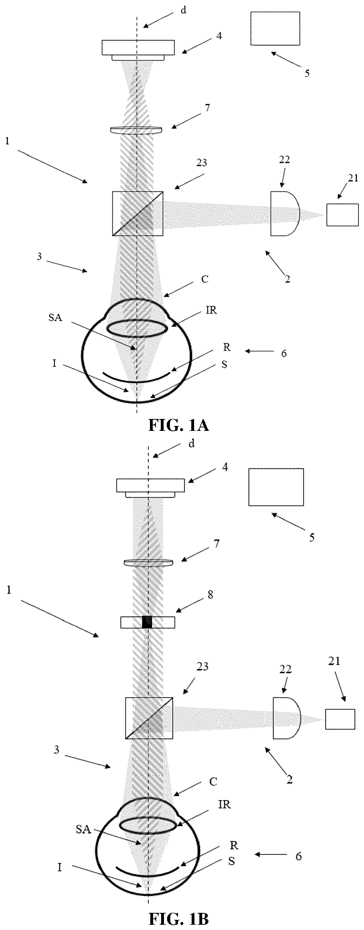

More precisely, the system 1 is a full-field transmission interference imaging system. Figures 1a and 1b represent example of the system 1 according to some embodiments referred to as retinal imaging embodiments. Full field is used here in opposition of imaging methods that use a scanning system to generate an image. Full field imaging uses an array of sensors such as those available in CCD or CMOS cameras. Illumination in the full-field systems is typically broader spatially (hundreds of micrometers - several millimeters) than that of scanning systems (typically, few microns).

As visible on Figure 1, the system 1 comprises an illumination arm 2, an object arm 3 and a detection arm. The detection arm comprises a bidimensional acquisition device 4. The system 1 also comprises a processing unit 5.

The object arm 3 is intended to receive, while in function, the eye 6 of the individual under examination. In function, the eye 6 of the individual is positioned in an imaging plane along the object arm 3. The object arm 3 presents an axis that is coincident with an imaging axis d. The axis of the object arm 3 and the imaging axis d do not necessarily need to overlap with the axis of the eye 6. For instance, the eye 6 can be tilted with respect to its axis.

The illumination arm 2 comprises a light source 21 positioned in a plane referred to as source plane, a first optical lens 22 and a polarization beam splitter 23. The light source 21 is typically chosen so as to emit spatially incoherent light to avoid speckle artifacts present in coherent sources. For instance, the light source 21 is a light emitting diode (LED) emitting in the near infrared (NIR) spectrum, around 850 nm. This wavelength is comfortable to be received by an eye due to the low sensitivity of the human eye at this wavelength. NIR light is also beneficial because: first, comparing to the visible light, NIR light is less absorbed in the choroid vessels underneath the retina, thus, more light remains available for the back-illumination; second, this wavelength is short enough for imaging with cellular resolution (of order of 1 micron); third, NIR light is efficiently detected by cameras based on CMOS technology, which are currently more developed than the technology of InGaAs cameras working with infrared light.

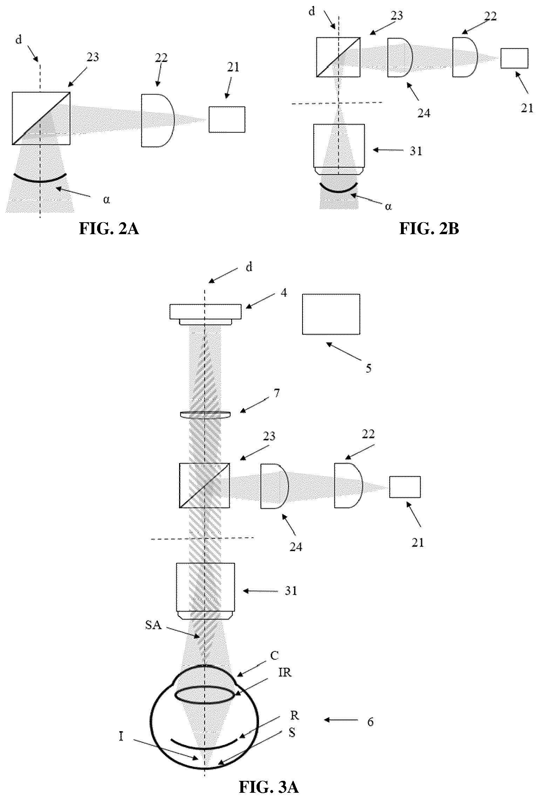

The light source 21, the first optical lens 22 and the polarization beam splitter 23 are coupled together to produce an illumination beam. The first optical lens 22 is chosen so that the illumination beam is substantially collimated. By substantially collimated, it is meant that the illumination beam presents a divergence angle $\alpha$ lower or equal to 20 degrees. As will be explained further, such a divergence angle $\alpha$ of the illumination beam will allow, when the system 1 is working with an eye 6 of an individual, to produce a substantially small light spot on an internal reflective layer of the eye 6. Figures 2a and 2b give an illustration of the divergence angle $\alpha$ for different embodiments of the system 1, namely respectively a retinal imaging embodiment and a corneal imaging embodiment. Let us take an example, when the internal reflective layer of the eye 6 is the sclera S of the posterior eye. The uncollimated illumination with the half-angle above 20 degrees will produce large illumination spot on the sclera above 6 mm (as calculated from the 17 mm focal length of the unaccommodated eye), on another hand the perfectly collimated beam will get focused by the eye into a minimal spot below 1 mm and whose size depends on the size of light source 21 and on the focal length of the first optical lens 22. In unaberrated unaccommodated eyes the minimal spot is located on the retina R overlaying the sclera S. In order to produce a minimal spot on the sclera S, the first optical lens 22 is slightly shifted along the illumination path producing a slightly divergent illumination (within 0-20 degrees) beam that can be efficiently focused on the sclera S. The range 0 to 20 degrees, specifically, is important to ensure high interference contrast in images, as will be seen later. The illumination beam is symmetric with respect to the axis of the object arm 3. The polarization beam splitter 23 is configured to polarize the illumination beam, reject the co-polarized specularly reflected light from the eye 6 and transmit the orthogonal polarization beam component after depolarization by the internal reflective layer.

When the system 1 is working, that is, when the eye 6 of the individual under examination is positioned in the imaging plane of the object arm 3, the illumination beam illuminates along the direction of the object arm 3 and penetrates the eye 6. Light is getting refracted by anterior eye, eventually forming a focal spot on an internal reflective layer of the eye 6. The choice of the internal reflective layer of the eye 6 and of the size of the focal spot on this layer is determined by the collimation degree of the illumination beam. For instance, the internal reflective layer of the eye 6 can be the retina R, the sclera S, the cornea C, or the iris IR, the most typical internal reflective layer being the sclera S.

In some embodiments, the system 1 further comprises a diaphragm positioned in the source plane. The diaphragm is configured to control and adjust the divergence angle of the illumination beam. As already mentioned, the collimation degree of the illumination beam impacts the contrast of the images obtained with the system 1.

The collimation degree can be controlled by the combination of optical elements in the system 1, including the first optical lens 22. For example, the required collimation can be achieved by either shifting the first optical lens 22 along the illumination arm 2 relatively to the light source 21 or by changing the focal length of the first optical lens 22 or by changing the size of the diaphragm, thus changing the effective size of the light source 21. The collimation illumination angle controlled by the effective light source size and the focal length of the first optical lens 22 can be written as: $\theta \simeq \dfrac{d}{F}$, where $\theta$ is the illumination angle, d is the effective size of the light source 21 determined by the actual size of the light source 21 and the openness of the diaphragm, F is the focal length of the first lens. For example, 1 mm LED source (typical size) not limited by the diaphragm and collimated by the first optical lens 22 with $F = 25$ mm, would produce the illumination beam I with about 2 degrees angular divergence.

The required collimation degree depending on the internal reflective layer of interest can be calculated as:

$$\alpha = \arctan\left[ \frac{p}{(1/f - 1/b)} \right]$$

, where $\alpha$ is the angle of the illumination, p is the half pupil size determining the NA of the eye 6 (in the dark $p = 3.5$ mm), f is the focal length of the unaccommodated eye (typically f = 17 mm), while b refers to the location of the internal reflective layer (for example, for sclera $b = 18$ mm). Therefore, the ideal angle of illumination required to focus on the sclera S is estimated around 1 degree. The size of the spot on the sclera S can be estimated from the formula: $s = 0.3 \cdot \alpha$, where $\alpha$ is the angle of the illumination, s is the diameter of the spot on the sclera S. Therefore, the spot on the sclera S is estimated around 0.3 mm. In reality the spot might be seen with extended Gaussian profile due to the extended size of the incoherent light source 21. As we will see later the size of the illumination spot would affect the interference contrast.

In another case of the iris IR being the internal reflective layer, the size of the illumination spot is controlled not by the focal length of the eye 6 but directly by the diaphragm.

Light is then back reflected by the internal reflective layer of the eye 6, forming incident light waves. The nature of the internal reflective layer of the eye 6 will depend on the embodiments and can be for instance the retina R, the sclera S, the cornea C, or the iris IR, as will be explained later.

Part of the incident light waves is directly retransmitted by the eye 6, forming a reference wave I. Another part of those incident light waves is scattered by a slice of the eye 6, forming a sample wave SA. The reference wave I and the sample wave SA have different divergence angles. The reference wave I and the sample wave SA produce together optical interferences. More specifically, the reference wave I is quasi-collimated. The collimation degree of the reference wave I is determined by the size of the focal spot on the internal reflective layer of the eye 6 and the focal length of the eye 6 as: $\beta \simeq \dfrac{s}{f}$, where $\beta$ is the angle of the reference wave I coming out of the eye 6, s is a focal spot on the internal reflective layer, f is the focal length of the eye. For example, the 1 mm beam spot focused on sclera of the unaccommodated eye with 17 mm focal length, would produce a quasi-collimated 3 degrees reference wave. On the opposite, the sample wave shows a broad divergence angle. More precisely, from the Mie theory of light it is known that the small organelles in the sample are scattering light into a large distribution of angles. For example (considering 850 nm illumination), 1 micron organelles scatter light in 50 degrees, 0.2 micron organelles scatter light in 100 degrees in forward direction. Both the quasi-collimated reference wave I and divergent sample wave SA are collected by the collection optical system - by either the optics of the eye 6 or by a third lens, as will be seen later, depending on the embodiment.

The difference in divergence angles between the reference wave and the sample wave is a key factor in producing high contrast interferences in the images acquired by the system 1. The quasi-collimated reference wave I fills only a small part of the numerical aperture (NA) of the collection optics, however the divergent sample wave SA fills the large portion of the NA. For example, the microscope objective with 0.3 NA has acceptance angle of 35 degrees - below the distribution of angles of the sample wave SA created by the sub-micron organelles. The sample wave SA is affected by the optical focus of the collection optical system and, thus exhibits an additional shift of optical phase, known as Gouy phase shift. The phase-shifted reference wave and sample wave produce interference with bright/dark contrast depending on the value of the phase shift. The phase is controlled by the NA of the collection optical system and the location of the organelle relatively to the imaging plane. Imaging plane coinciding with the optical focal plane. The above explains the requirement of collimated illumination and small spot on the internal reflective layer: the smaller is the focusing spot, the more collimated is the reference wave, then the less the reference wave is affected by the Gouy phase shift, leading to the larger phase shift between the sample and reference waves and higher interference contrast. Typically, the optimal image contrast is obtained when the illumination beam has a divergence angle of about 4 degrees, corresponding to a spot size on the internal reflective layer of 1 millimeter. The total beam formed by both the reference wave I and the sample wave SA is referred to as output beam in what follows.

As represented on Figure 1, the reference wave I and the sample wave SA impinge on the polarization beam splitter 23. The polarization beam splitter 23 is also configured to discriminate the transmission light of interest from the reflected light. More precisely, the specularly reflected light from the surface of the eye 6 keeps the initial polarization and, therefore is filtered out (reflected from the detection) by the same beam splitter 23 on the backward pass. On another hand, light reflected from the internally multi-diffusely reflective layer, such as the sclera S, becomes depolarized, then after transmission/refraction through the sample layers this light can pass through the polarization beam splitter 23 and can be detected. Additionally, a linear polarizer can be added between the polarization beam splitter 23 and the light source 21 or between the polarization beam splitter 23 and the bidimensional acquisition device 4 to improve the polarization selection of the polarization beam splitter 23 (for example, the typical polarization extinction ratio of the beam splitter is 1000:1 and worsens for non-collimated light beams, while the extinction ratio of the linear polarizers can reach 100 000:1). Indeed, after specular reflection, this light is no more polarized, thus is not transmitted through the polarization beam splitter 23.

The bidimensional acquisition device 4 is positioned along the imaging axis and comprises a plurality of sensors arranged in a detection plane. The detection plane is optically conjugated with the imaging plane of the object arm 3, by an optical system OS comprising the polarization beam splitter 23 and a tube lens 7. Depending on the embodiments, the optical system OS comprises additional elements that will be further described. When the system 1 is working, light coming from the optical interferences between the reference wave I and the sample wave SA impinges on the plurality of sensors, which acquire a corresponding bidimensional interferometric signal. In what follows, the expression "bidimensional interferometric signal" will be used indifferently with the expression "interferometric image".

The tube lens 7 is part of the optical system OS conjugating the imaging plane with the detection plane. It is then also possible with the system 1 to acquire bidimensional interferometric signals representative of the retina R of the eye 6 of the individual. The tube lens 7 is configured to adjust an optical magnification of interferometric images acquired by the bidimensional acquisition device 4, so as to optimize the size of patterns observed in the bidimensional interferometric signals acquired by the bidimensional acquisition device 4. Typically, the size of the patterns is chosen based on the resolution of the optical system OS and size of the bidimensional acquisition device 4 to ensure patterns sampling according to the Nyquist signal sampling criterion.

For instance, the bidimensional acquisition device 4 is a CMOS camera with enhanced sensitivity to near infrared light (for instance with a quantum efficiency of about 25% to 50% at 850 nm). The camera frame rate typically allows acquiring images at least at 80 frames per second so as to suppress the influence of the movements of the eye 6 of the individual under examination.

The processing unit 5 is configured to process the bidimensional interferometric signals acquired by the bidimensional acquisition device 4.

In some embodiments, the system 1 further comprises a linear polarizer positioned between the polarization beam splitter and the light source or between the polarization beam splitter and the bidimensional acquisition device, or at both of those positions. Such a linear polarizer is meant to improve the polarization degree of the illumination beam, when it is positioned between the polarization beam splitter 23 and the light source 21. Improving the polarization degree of the illumination beam helps improving the contrast of the interferometric images obtained with the system 1. When the linear polarizer is positioned between the polarization beam splitter and the bidimensional acquisition device, the linear polarizer is meant to further reject the parasitic stray light specularly reflected by the eye 6 of the individual under examination.

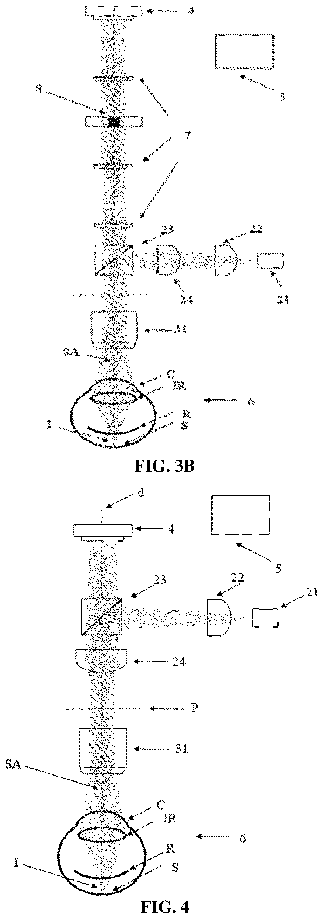

In some embodiments referred to as anterior eye imaging embodiments, and as illustrated in Figure 3, the system 1 further comprises a second optical lens 24 and a third optical lens 31. Those embodiments correspond to cases where the system 1 produces an image of the cornea C or crystalline lens (LC) of the eye 6. The second optical lens 24 is configured to produce from the illumination beam a focused beam focusing in a back focal plane P of the third optical lens 31, so that a substantially collimated beam comes out of the third optical lens 31. The third optical lens 31 is part of the optical system S conjugating the imaging plane with the detection plane. The second optical lens 24 may also be part of the optical system OS conjugating the imaging plane with the detection plane, if it is implemented in the object arm 3, as illustrated on Figure 4. In those cases, the system 1 is more compact.

For example, the third lens 31 is a microscope objective, a turret of microscope objectives or a zoom microscope objective, these objectives having numerical aperture of 0.25 or higher. Typically, when the third lens 31 is a microscope objective, it has 10X magnification, 0.3 NA (numerical aperture) and 18mm working distance or 20X magnification, 0.45 NA and 8 mm working distance or 50X magnification, 0.65 NA and 10mm working distance. These values of working distance are advantageous for the safety of the eye 6 of the individual under examination. However, objectives with other magnifications, numerical apertures and working distances can also be used. Also, the third lens 31 may be a conventional optical lens.

Embodiments where the system 1 does not comprise the third optical lens 31 are referred to as retinal imaging embodiments.

It is also possible with the system 1 to acquire series of depth wise interferometric images of some portion of the eye 6 of the individual under examination. To this aim, in some embodiments, the system 1 comprises either a moving element, or a controllable element.

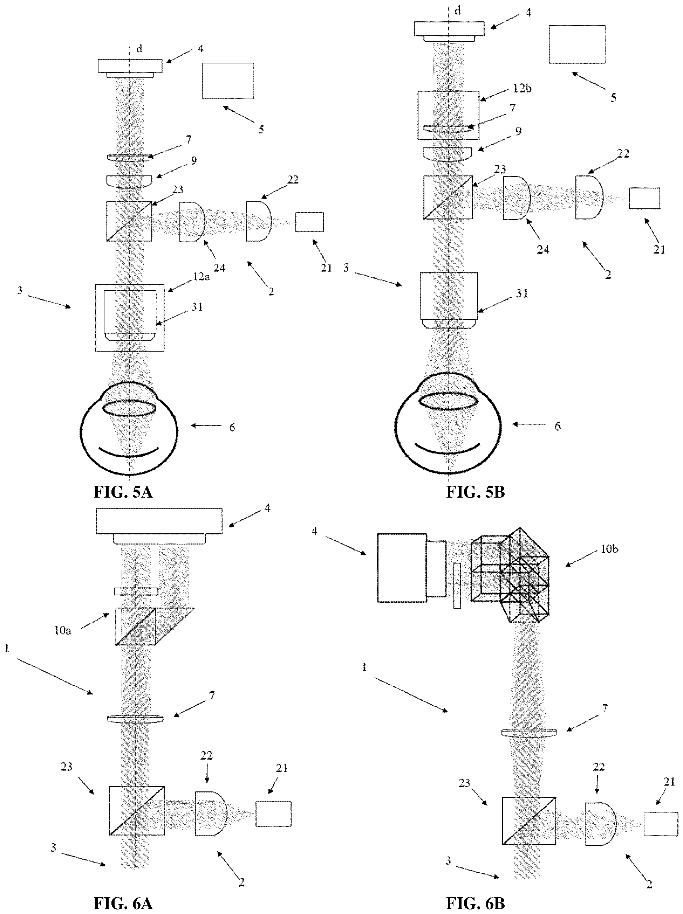

For instance, in some embodiments, the system 1 comprises between the polarization beam splitter and the bidimensional acquisition device a tunable lens 9. More precisely, the tunable lens 9 is part of the optical system OS conjugating the imaging plane with the detection plane. The tunable lens 9 has a focal length controllable with the processing unit. By tuning the focal length of the tunable lens 9, the imaging plane on the object arm 3 is adjusted. Figure 5 shows example of the system 1 comprising a tunable lens 9.

In some embodiments, when the system 1 comprises a third lens 31, the third lens 31 is mounted on a moving platform 12a controllable with the processing unit. For instance, the moving platform 12a moves thanks to a piezoelectric or voice coil motor. When moving the moving platform, the imaging plane on the object arm 3 is adjusted. An example of moving platform 12a is illustrated on Figure 5a.

In other embodiments, the tube lens 7 is mounted on a moving platform 12b controllable with the processing unit 5. For instance, the moving platform moves thanks to a piezoelectric or voice coil motor. When moving the moving platform 12b, the imaging plane on the object arm 3 is adjusted. An example of moving platform 12b is illustrated on Figure 5b.

It is also possible with the system 1 to acquire simultaneously several interferometric images, with an application to optical sectioning as will be described later. To this aim, in some embodiments the system 1 comprises an optical multiplexing system 10a, 10b positioned between the polarization beam splitter 23 and the bidimensional acquisition device 4.

For instance, the optical multiplexing system 10a, 10b comprises a plurality of additional beam splitters or prisms, or glass plates spatially shifted with respect to the polarization beam splitter 23. For instance, as illustrated on Figure 6a, there are two additional beam splitters receiving a portion of the output beam. The optical path travelled by the part of the output beam received by the additional beam splitter is different from the optical path travelled by the part of the output beam reflected by the two additional beam splitters. Therefore, two bidimensional interferometric signals with different optical phases are acquired by the bidimensional acquisition device. The two parts of the bidimensional acquisition device 4 are conjugated to two different imaging planes. Close-to-$\pi$ phase difference between the reference and sample waves is adjusted by changing the defocus of each beam via the thicknesses of the beam splitters and, optionally, glass plates. The dimensions of those components are determined by the dimensions of the bidimensional acquisition device, and magnification of the optical system OS. Magnification is important because the axial position of the image plane in the sample varies with the magnification squared in the plane of the bidimensional acquisition device 4. For example, one optimal configuration to support a 11 mm sensor and 0.3 NA and 10X magnification optics would be a 5 mm beam splitter, a 5 mm mirror and a 5 $\pm$ 1mm compensation glass, such configuration allowing to simultaneously capture two image planes axially separated by 10 microns.

Figure 6b shows another example, where four bidimensional interferometric signals with four different optical phase differences are acquired simultaneously by the bidimensional acquisition device 4. In this case, the optical multiplexing system 10b is composed of a combination of four beam splitters and, optionally, glass plates. This configuration consists of a first beam splitter that divides the light beam into two paths. For each path, a beam splitter and a right-angle prism are placed respectively to divide a portion of the incoming beam from the tube lens into four light beams, equally distributed in terms of optical power light. The relative optical phase shift between the beams can be adjusted by implementing the glass plates of different thickness in each optical path.

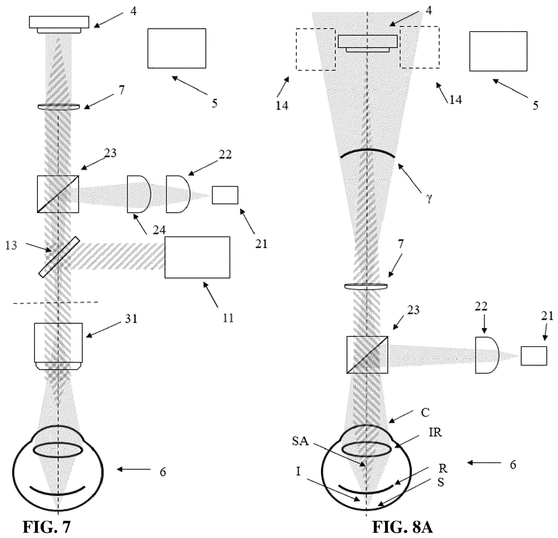

In some embodiments, the system 1 is coupled to an additional imaging system 11, as illustrated on Figure 7.

For instance, the system 1 further comprises an additional eye imaging system, such as a macro camera, a stereo camera, a pupil camera, a fundus camera system, a slit lamp system or an optical coherence tomography system. The additional eye imaging system is coupled to the system 1 by means of a dichroic mirror or glass plate 13 placed in the illumination arm 2, in the object arm 3 or between the polarization beam splitter 23 and the bidimensional acquisition device 4. Typically, the additional system provides the global view of the eye with larger field of view than the transmission interferometric device 1. This allows for easy aligning to the eye as well as qualitative general health examination. In addition, such a device allows to register lateral eye movements during the acquisition, allowing to have feedback for the reconstruction of the images.

In another example, the additional imaging system 11 is an axial position tracking system. By axial position tracking system, it is meant a system that can estimate the axial position of one of the layers of the eye 6 relatively to the system 1. One example of such axial position tracking system is an optical coherence tomography system. The additional axial position tracking system is coupled to the system 1 by means of a dichroic mirror placed in the illumination arm 2, in the object arm 3 or between the beam splitter 23 and the bidimensional acquisition device 4. Additionally, axial position tracking system can be made with: a macro camera or a pupil camera through the estimation of the defocus in the image, or with a stereo camera.

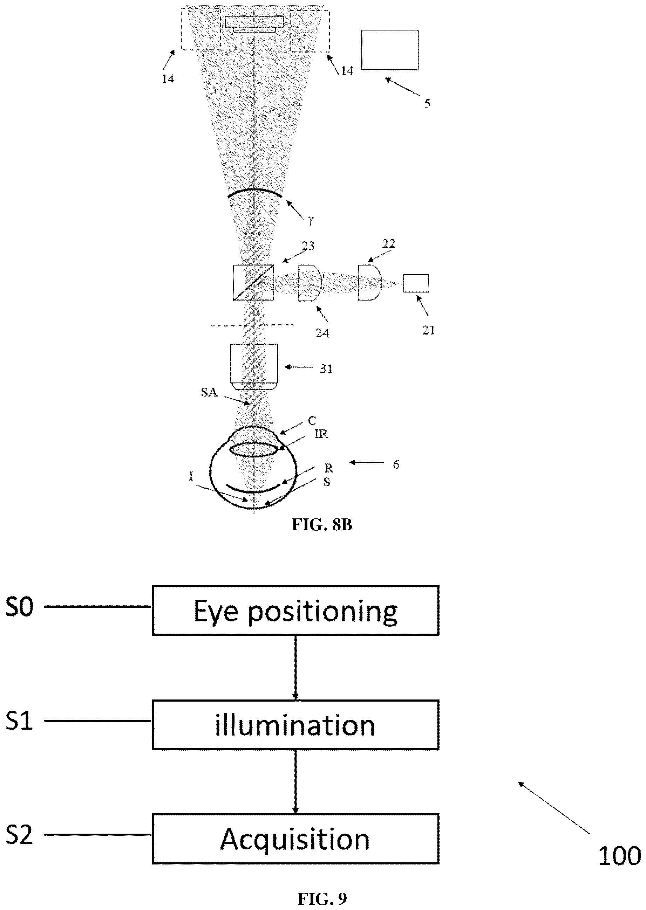

In some embodiments, the detection arm presents a relatively long length. In other words, the distance between the polarization beam splitter 23 and the bidimensional acquisition device 4 is relatively long. Examples of those embodiments are illustrated in Figures 8a and 8b, corresponding respectively to a retinal imaging embodiment and to a corneal imaging embodiment. In those embodiments, the value of the divergence angle $\gamma$ of the reference wave after transmission through the polarization beam splitter 23 towards the bidimensional acquisition device 4 is such that large portions 14, schematically represented by dotted rectangles, of the reference wave do not reach the bidimensional acquisition device 4, therefore reducing the amount of light from the reference wave in the total signal received by the bidimensional acquisition device 4. The reference wave is typically larger than the sample wave, thus reducing the sample wave balances the optical interferometer and improves the contrast. In those embodiments, the typical value of the distance between the polarization beam splitter 23 and the bidimensional acquisition device 4 is between 250 mm and 700 mm.

Now, a method 100 for in vivo, full-field transmission interference imaging of an eye of an individual will be described. The method is implemented with a system 1 as previously described. Figure 9 is an example of a flow chart describing the method 100.

In a step S0, the individual under examination is placed so that the eye 6 to be examined is disposed at an imaging plane of the object arm 3 of the system 1.

In a step S1, the eye 6 is illuminated with the illumination beam. The illumination beam penetrates the eye 6, focuses onto an area of an internal reflective layer of the eye, and is backreflected by the internal reflective layer of the eye. In the case the system 1 is a system according to the retinal imaging embodiments, the internal reflective layer is the retina R of the eye 6. In the case the system 1 is a system according to the corneal imaging embodiments, the internal reflective layer is either the sclera S of the eye 6 or the iris IR of the eye 6. The backreflected light forms incident light waves. As previously described, a reference wave exits the eye after the incident light waves have been transmitted by the eye, and a sample wave exits the eye after the incident light waves have been scattered by a voxel of a slice of the eye 6 of the individual.

In a step S2, the bidimensional acquisition device 4 acquires a bidimensional interferometric signal, or in other words, an interferometric image.

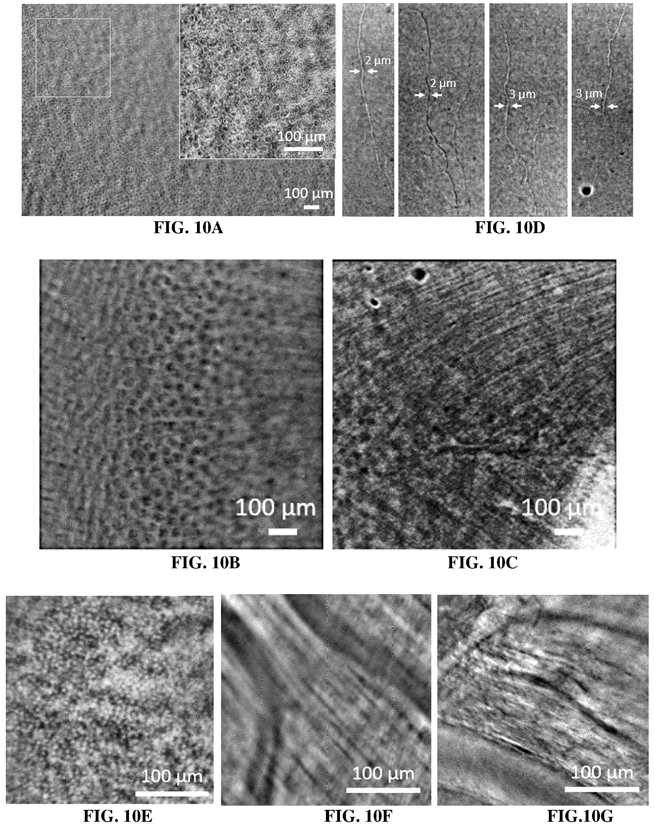

Figure 10a, 10b, 10c, 10d, 10e, 10f and 10g show respectively images obtained with the method 100 of: corneal endothelial cells; the epithelium of a crystalline lens, fibers of a crystalline lens; corneal nerves; retinal photoreceptors; nerve fibers of a retina; blood vessels of a retina.

In some embodiments, the method 100 for in vivo, full-field transmission interference imaging of an eye 6 of an individual allows obtaining three-dimensional images of a volume of the eye 6 under examination. In those embodiments, the method is implemented with the system 1 when the system 1 comprises a moving component or a controllable component. In the case of a moving component, the moving component is moved so as to displace the imaging plane along the imaging axis in order to acquire a plurality of bidimensional interferometric signals at different working planes along the imaging axis. In the case of a controllable component, i.e., a component with a variable characteristic, the variable characteristic is varied so as to displace the imaging plane along the imaging axis in order to acquire a plurality of bidimensional interferometric signals at different working planes along the imaging axis.

For instance, when the system 1 comprises a tunable lens 9, the processing unit 5 controls the focal length of the tunable lens 9.

In another example, when the system 1 comprises a third lens 31 mounted on a moving platform controllable with the processing unit 5, the processing unit 5 controls the movement of the moving platform so as to vary the total focal length of the optical system, thereby moving the imaging plane.

In another example, when the system 1 comprises a tube lens 7 mounted on a moving platform controllable with the processing unit 5, the processing unit 5 controls the movement of the moving platform so as to vary the total focal length of the optical system OS, thereby moving the imaging plane.

In all these examples, a three-dimensional image of a volume of the eye 6 is obtained by gathering all images acquired for the different positions of the imaging plane, from which a volumetric three-dimension view of the volume is obtained.

More specifically, one can vary the position of the imaging plane across several millimeters along the imaging axis. During this on-going focus swipe the sequence of images can be acquired with each image being captured from the different depths of the eye 6. If the focus swipe and the image acquisition are fast (a few seconds), the eye 6 remains almost static during the entire acquisition. Thus, only small realigning in post-processing is needed to reconstruct the three-dimensional image based on the sequence of images.

In some embodiments, the method 100 allows for optical sectioning. Optical sectioning means imaging in a given plane, or in other words, in a given section, with rejection of out-of-focus light. In those embodiments, either the method is implemented with the system 1 when the system 1 comprises a moving component or a controllable component, or the system 1 comprises an optical multiplexing system 10a, 10b.

In the case of a moving component, the moving component is moved so as to displace the imaging plane along the imaging axis so as to acquire a plurality of bidimensional interferometric signals at different imaging planes along the imaging axis. In the case of a controllable component, i.e., a component with a variable characteristic, the variable characteristic is varied so as to displace the imaging plane along the imaging axis in order to acquire a plurality of bidimensional interferometric signals at different imaging planes. In the case of an optical multiplexing system 10a, 10b, different bidimensional interferometric signals are acquired simultaneously.

A plurality of bidimensional interferometric signals are acquired by the bidimensional acquisition device 4 for imaging planes located within a depth in the vicinity of the given section (or from different beams received via the optical multiplexing system 10a, 10b) corresponding to optical phase differences comprised between $-\pi/2$ and $+\pi/2$.

Then, change in the optical phase difference affects the visible intensity of the light detected by the bidimensional acquisition device 4. This can be illustrated with a Gaussian model for which the electric fields of the transmitted and scattered waves can be expressed as:

$$\begin{cases} E_S \sim exp\left(-i \cdot \left(k \cdot z + \underbrace{\frac{k \cdot r^2}{2 \cdot R(z)}}_{defocus} - \underbrace{arctan\left(\frac{z}{z_0}\right)}_{Gouy\ phase} - \frac{\pi}{2}\right)\right), \\ E_T \sim exp(-i \cdot k \cdot z) \end{cases}$$

where: $E_S$, $E_T$ are the electric fields of the scattered and transmitted waves respectively, $k$ is the wavenumber, r and z are respectively the lateral and axial distances from the optical focus (r = 0, z = 0 at the focus), R(z) is the radius of curvature of the beam's wavefront at z, $z_0$ is the Rayleigh length, equivalent to the half depth-of-field. The two waves interfere producing intensity detected by the camera: $I=|E_T|^2+|E_S|^2+ 2 \cdot RE\{E_S \cdot E_T{}^*\}$, where RE denotes the real part of the complex number.

Combining the terms that are constant with z, the equation can be rewritten:

$$I \sim I_0 \cdot \left(1 + \frac{z/z_0}{1+\left(z/z_0\right)^2}\right)$$

, where $I_0$ is constant intensity of non-interfering light. The second term comes from the interference and changes the sign around the optical focus. Near the focus where $z/z_0 \ll 1$ the equation simplifies to $I \sim I_0 \cdot (1+z/z_0)$.

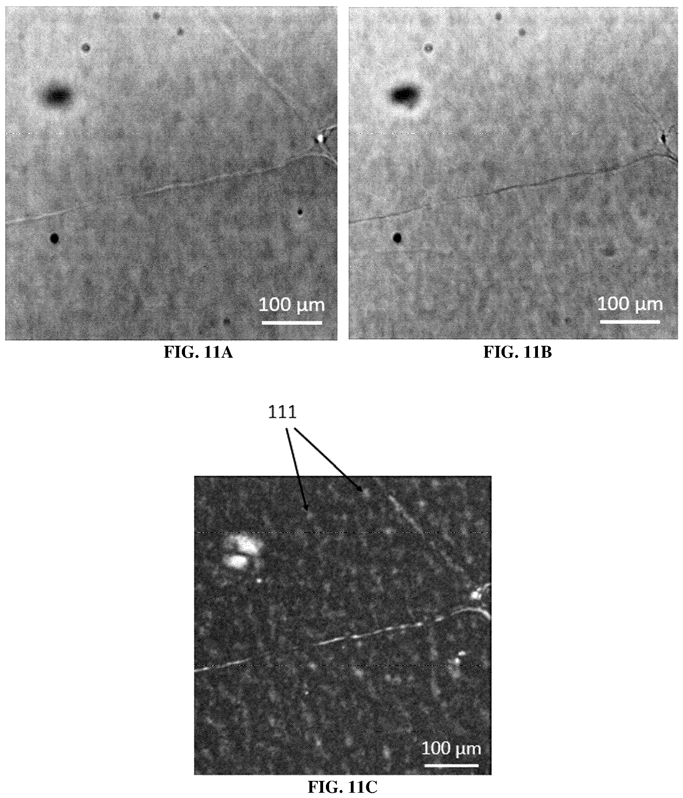

From at least two two-dimensional interferometric signals acquired by moving the imaging plane (or acquired via the optical multiplexing system), and thus the phase of the interferometric signal, it is possible to reject, by image processing, the out of focus light and retrieve an image of the optical section, for example by pixel by pixel difference between the two acquired interferometric signals. Figures 11a and 11b show two interferometric images of the corneal stroma, corresponding to two different phases that, when one is subtracted from the other, give an optical section shown on Figure 11c. Bright keratocyte cells 111 can be observed in the optical section of Figure 11c.

More specifically, by acquiring two dimensional interferometric signals for different imaging planes, and by computing the difference between the two bidimensional interferometric signals, the following image intensity is obtained:

$$I_1 - I_2 = \underbrace{I_0 - I_0}_{\substack{out-of-focus \\ non-interfering light}} + \frac{z_2 - z_1}{z_0} = \frac{\Delta z}{z_0}$$

Therefore, from equation in [0091] it follows that, if an object (such as a cell) is located far from the imaging plane, the intensity difference is zero and the signal is filtered out from detection. However, if an object that scatters light is close to the imaging plane, the intensity obtained by difference is non zero and getting enhanced. This process is referred to as optical sectioning and tomography.

In some embodiments, the method 100 for in vivo, full-field transmission interference imaging of an eye 6 of an individual allows obtaining three-dimensional images of a volume of the eye 6 under examination from images obtained by optical sectioning as previously described. The method includes moving the imaging planes so as to successively compute a plurality of section images, and then the gathering of the plurality of the section images.

It is possible to enhance the contrast of the interferometric image acquired at step S4.

Indeed, for example, previously to step S2, a plurality of interferometric images are acquired, each at a distinct timepoint. An image, referred to as average image, is computed by the processing unit 5. For example, the average image is a linear combination of the plurality of the interferometric images. Then at step S4, an effective image at a timepoint subsequent to the distinct timepoints, of which the contrast is to be improved. In a step S5, the processing unit computes a subtraction of the effective image and the computed average image. The result of the subtraction is an image with improved contrast, where the static background light has been removed from the scene.

It is possible to enhance the signal to noise ratio of the interferometric image acquired at step S2.

Indeed, for example, previously to step S2, a step of acquiring at least one training set of images of a training sample, each training set including a first training image of a section of the training sample and a second training image of a same section of the training sample obtained as a time average image of several images. The target average image typically has a higher signal-to-noise ratio, larger bit depth (i.e. dynamic range) and smaller movement artefacts. Training images are acquired from the in vivo human eye (cornea, crystalline lens, retina). After the acquisition of the at least one training set of images, a step of training an artificial neural network is performed. This step comprises, for a given training set of images, providing the corresponding first training image to said artificial neural network as an input, and providing the corresponding second training image as a target output of the artificial neural network. An image-to-image transformation network, typically a U-net network, can be used. The application of this step to applications of the present invention is unique because the structures in the images obtained with the method 100 can be seen with the opposite contrasts (phases), i.e., bright or dark, depending on the axial position of the tissue relative to the system 1. The second training image is typically obtained first by acquiring images with a reduced camera field of view, so as to increase the imaging speed and suppress movement artifacts. Typically, images with high bit depth (dynamic range), 12 bit and higher, are acquired. The second training image is then obtained by averaging the acquired time stack of single images to increase signal to noise ratio. The first training image is selected as one of the images from the same stack. Typically, an additional Gaussian noise is added to the first image to simulate the low-signal-to-noise cameras. The trained neural network can then learn the statistical patterns of converting a first image obtained at step d) of the method 100 into a clean and denoised second image.

After the acquisition, at step S4, of the interferometric image, the method further comprises inputting the acquired interferometric image to the trained artificial neural network. The output of the trained artificial neural network is a denoised image.

The method 100 allows for several applications.

For instance, the method 100 improves image quality to allow for visualizing cells and counting a number of cells in an interferometric image acquired at step S4. In this case, the method 100 comprises after step S4 a step S6, implemented by the processing unit 5, of segmenting and counting cells and nerves within the interferometric image. For instance, a U-net neural network can be used for segmentation.

In another example, the method 100 allows for axial tracking of the eye of the individual under examination. In this case, the method is implemented with the system 1, wherein the system 1 is coupled to an axial position tracking system, for instance an optical coherence tomography system. The axial position tracking system is coupled to the system 1 by means of a dichroic mirror or a glass plate 13 in the illumination arm 2, in the object arm 3 or between the polarization beam splitter 23 and the bidimensional acquisition device 4. In this case, the method further comprises, at step S4, obtaining at least one en-face image with the system 1 at at least one timepoint. An en-face image shows a particular layer or section of a tissue being imaged. Then, for each among the at least one en-face image, the method further comprises acquiring at least one corresponding image with the optical coherence tomography device. Finally, the method further comprises computing, based on the at least one corresponding image acquired with the additional optical coherence tomography image an axial position of the eye of the individual along the imaging axis over time.

For instance, we can consider the case where the axial position tracking system is an optical coherence tomography system 11 (OCT). By OCT, it is typically meant spectral domain OCT or swept-source OCT, by which cross-sectional views are acquired. Some layers corresponding to cross-sectional views are preselected (such as the corneal surface, the retinal nerve fiber layer) are segmented. Then, the position of the segmented layers relatively to the center of the image is calculated.

Other devices can be used as axial position tracking system, such as a macro camera, a pupil camera and a slit lamp system. Those devices can acquire images that can be processed. The depth, or axial position, can be retrieved by calculating the defocus of the images. A stereo camera can be used to retrieve the depth directly from the two stereo images.

In another example, the method 100 allows for imaging cell dynamics of an eye 6 of an individual. In this case, the method 100 further comprises computing, using the processing unit of the system 1, an image referred to as dynamics image. The dynamics image is representative of temporal variations of intensity between dimensional interferometric signals among the set of bidimensional interferometric signals. More precisely, the temporal fluctuations are acquired in each pixel. The average offset background is removed from each image to reveal temporal intensity fluctuations due to the moving organelles in each voxel in the imaging plane. Those intensity fluctuations are then presented in color scale to describe the fluctuation characteristics of each pixel such as, the spectrum mean value and bandwidth of the Fourier Transform and, the standard deviation of the fluctuation. Encoding those values in the RGB or HSV colormap produces colorful dynamic image, where the brighter color denotes the faster organelles movements.

Variants are also possible.

In some variants, the system 1 further comprises an optical mask 8. Those variants are for instance illustrated on Figures 1b and 3b. The optical mask is positioned in a plane referred to as mask plane, located between the polarization beam splitter 23 and the bidimensional acquisition device 4. The system 1 further comprises a lens or a system of lenses conjugating the back focal plane of the third optical lens 31 with the mask plane. The optical mask 8 is configured to block the portion of the reference wave. As a consequence, the contrast of the images acquired by the bidimensional acquisition device 4 is improved due to the balancing of the sample wave and reference wave in the interferometer.

**Claims**

1. A system (1) for in vivo, full-field transmission interference cellular resolution imaging of an eye (6) of an individual, said eye having an outer surface, the system (1) comprising:

   - an object arm (3) intended to receive, when the system (1) is in operation, the eye (6) of the individual in an imaging plane, said object arm (3) having an axis coincident with an imaging axis,
   - an illumination arm (2) comprising a light source (21) coupled to a first optical lens (22) and to a polarization beam splitter (23), configured to produce together an illumination beam, wherein the illumination beam is substantially collimated and symmetric with respect to the axis of the object arm (3),

      wherein, in operation, at least part of the illumination beam penetrates the eye (6) of the individual, focuses onto an area of an internal reflective layer of the eye (6), and is backreflected by said internal reflective layer to form incident light waves,
      wherein, in operation, said imaging plane is located between said internal reflective layer of the eye (6) and said outer surface of the eye (6),

   - a detection arm comprising a tube lens (7) and a bidimensional acquisition device (4), said bidimensional acquisition device (4) comprising a plurality of sensors arranged in a detection plane, configured to acquire at least one bidimensional interferometric signal resulting from interferences between a substantially collimated reference wave obtained by transmission of said incident light waves and a divergent sample wave obtained by scattering said incident waves by a voxel of a slice of the eye (6) of the individual, said detection plane being optically conjugated with said imaging plane by an optical system (S) comprising said polarization beam splitter (23),

      wherein the divergent sample wave presents an additional optical phase shift relatively to the substantially collimated reference wave,
      wherein, the polarization beam splitter (23) is configured to polarize the illumination beam and to filter out, in operation, light specularly reflected by the eye (6) of the individual.

2. The system (1) according to claim **1,** wherein the illumination beam presents a divergence angle of less than 20 degrees, said divergence angle being measured with respect to the axis of the object arm (3), and wherein one dimension of said area of the internal reflective layer, when illuminated by the illumination beam, is less than 6 millimeters.

3. The system (1) according to claim **1** to **2,** wherein the light source (21) is positioned in a source plane, the system (1) further comprising an aperture positioned in said source plane, said aperture being configured to control the divergence angle of the illumination beam.

4. The system (1) according to claim **1** to **3,** further comprising a processing unit (5) and a tunable lens (9) positioned between the polarization beam splitter (23) and the bidimensional acquisition device (4), said tunable lens (9) having a controllable focal length and being configured to be controlled so as to acquire a plurality of bidimensional interferometric signals to determine a plurality of working images, each among the plurality of working images corresponding to a distinct imaging plane along the imaging axis, wherein said processing unit (5) is configured to compute a 3D image of the eye (6) of the individual based on the plurality of working images.

5. The system (1) according to claim **4,** wherein the controlling of said tunable lens focal length, so as acquire a plurality of bidimensional interferometric signals depth wise inside a section of the eye of the individual, results in a plurality of predetermined phase differences between the reference wave and the sample wave comprised between -π/2 and +π/2, and wherein the processing unit (5) is further configured to compute a sectioning image, based on a linear combination of said bidimensional interferometric signals among the plurality of bidimensional interferometric signals.

6. The system (1) according to claims **1** to **5,** further comprising an optical multiplexing system (10a, 10b) positioned between the polarization beam splitter (23) and the bidimensional acquisition device (4), so as to acquire in a single acquisition a plurality of bidimensional interferometric signals depth wise inside a section of the eye (6) of the individual, said optical multiplexing system (10a,10b) being configured to provide predetermined phase differences between the reference wave and the sample wave comprised between -π/2 and +π/2.

7. The system (1) according to any of claims **1** to **6,** further comprising a second optical lens (24) positioned in the illumination arm (2) or in the object arm (3), wherein:

   - said object arm (3) further comprises a third optical lens (31),
   - said second optical lens (24) is configured to produce a focused beam from the illumination beam so that said focused beam focuses in a back focal plane of said third optical lens (31), so that a substantially collimated beam comes out of the third optical lens (31).

8. The system (1) according to claim 7, wherein the third optical lens (31) is a microscope objective, a turret of microscope objectives or a zoom microscope objective, said objectives having a numerical aperture of 0.25 or higher.

9. The system (1) according to claims **7** or **8,** further comprising an optical mask (8) placed in a plane referred to as mask plane between the polarization beam splitter (23) and the bidimensional acquisition device (4), and a lens or a system of lenses conjugating the back focal plane of the third lens (31) with the mask plane, said optical mask (8) being configured to partially block part of the reference wave, so as to improve image contrast.

10. The system (1) according to claims **7** to **9,** further comprising a moving platform on which said third optical lens (31) is mounted and a processing unit (5), said moving platform being configured to move along the imaging axis so as to acquire a plurality of bidimensional interferometric signals to determine a plurality of working images, each among the plurality of working images corresponding to a distinct imaging plane along the axis of the object arm (3), and wherein said processing unit (5) is configured to, when moving said platform along said an imaging axis, compute a 3D image of the eye (6) of the individual based on the plurality of working images.

11. The system (1) according to claim **10,** wherein moving said moving platform along said imaging axis, so as acquire the plurality of bidimensional interferometric signals depth wise inside a section of the eye (6) of the individual, results in a plurality of predetermined phase differences between the reference wave and the sample wave comprised between -π/2 and +π/2, and wherein the processing unit (5) is further configured to compute a sectioning image, based on a linear combination of said bidimensional interferometric signals among the plurality of bidimensional interferometric signals.

12. The system according to claims **1** to **11,** further comprising an additional eye imaging system, such as a fundus camera system or a slit lamp system, or an additional position tracking system, such as a macro view camera system, a stereo camera, a pupil camera, said additional eye imaging system or said additional position tracking system being integrated by means of a dichroic mirror placed in the illumination arm (2), in the object arm (3) or between the polarization beam splitter (23) and the bidimensional acquisition device (4).

13. A method (100) for in vivo, full-field transmission interference imaging of an eye (6) of an individual, said method comprising:

   a) disposing said eye (6) at an imaging plane in an object arm of a system (1) for in vivo, full-field transmission interference imaging,
   b) illuminating with a substantially collimated, spatially incoherent and symmetric illumination beam said eye (6) of the individual so that said illumination beam focuses on a substantially small area of an internal reflective layer of the eye (6) and is back reflected by said internal reflective layer to form incident light waves,
   c) acquiring, by a dimensional acquisition device (4) comprising a plurality of sensors arranged in a detection plane, at least one bidimensional interferometric signal,

said at least one bidimensional interferometric signal resulting from interferences between a reference wave obtained by transmission of said incident light waves, and a sample wave obtained by scattering said incident light waves by a voxel of a slice of the eye (6) of the individual,
said imaging plane being conjugated with said detection plane,

d) obtaining at least one first image from said at least one bidimensional interferometric signal by means of a processing unit (5) of said system (1).

**14.** The method (100) according to claim **13,** wherein the system for in vivo, full-field transmission interference imaging is a system according to any of claims **1** to **12.**

**15.** The method (100) according to claim **13,** wherein the system for in vivo, full-field transmission interference imaging is a system according to claim 12 when it further comprises an additional position tracking system and , at step d), at least one en-face image is obtained with the system for in vivo, full-field transmission interference imaging at at least one timepoint, the method further comprising:

- for each among the at least one en-face image, acquiring at least one corresponding image with the additional position tracking system;
- computing, based on the at least one corresponding image acquired with the additional position tracking system an axial position of the eye (6) of the individual along the imaging axis over time.

**16.** The method (100) according to any of claims **13** to **15,** wherein, at step c), a set of bidimensional interferometric signals is acquired,
the method further comprising computing, using the processing unit (5) of the system (1) for in vivo, full-field transmission interference imaging, an image referred to as dynamics image, said dynamics image being representative of temporal variations of intensity between said bidimensional interferometric signals among the set of bidimensional interferometric signals.

FIG. 1A

FIG. 1B

FIG. 2A

FIG. 2B

FIG. 3A

**FIG. 3B**

**FIG. 4**

FIG. 5A

FIG. 5B

FIG. 6A

FIG. 6B

**FIG. 7**

**FIG. 8A**

**FIG. 8B**

SO — Eye positioning

S1 — illumination

S2 — Acquisition

100

**FIG. 9**

**FIG. 10A**

**FIG. 10D**

**FIG. 10B**

**FIG. 10C**

**FIG. 10E**

**FIG. 10F**

**FIG.10G**

FIG. 11A

FIG. 11B

FIG. 11C

Europäisches
Patentamt

European
Patent Office

Office européen
des brevets

# EUROPEAN SEARCH REPORT

Application Number

EP 23 17 1187

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/153904 A1 (MUKOH MASAKI [JP] ET AL) 2 June 2016 (2016-06-02) | 1-3, 5-11,13, 14,16 | INV. A61B3/10 A61B3/12 |
| Y | * the whole document * | 4,12,15 | A61B3/13 |
| Y | WO 2022/162224 A1 (ZEISS CARL MEDITEC INC [US]; ZEISS CARL MEDITEC AG [DE]) 4 August 2022 (2022-08-04) * paragraph [0104] * * paragraph [0120] * | 4,12,15 | |
| A | US 2017/059299 A1 (SAFRANI AVNER [IL] ET AL) 2 March 2017 (2017-03-02) * paragraph [0004] - paragraph [0006] * * paragraph [0082] - paragraph [0084] * * paragraph [0098] * | 1-16 | |

TECHNICAL FIELDS SEARCHED (IPC)

A61B

The present search report has been drawn up for all claims

| Place of search | Date of completion of the search | Examiner |
|---|---|---|
| Munich | 20 September 2023 | Alvazzi Delfrate, S |

EPO FORM 1503 03.82 (P04C01)

## ANNEX TO THE EUROPEAN SEARCH REPORT
## ON EUROPEAN PATENT APPLICATION NO.

EP 23 17 1187

This annex lists the patent family members relating to the patent documents cited in the above-mentioned European search report.
The members are as contained in the European Patent Office EDP file on
The European Patent Office is in no way liable for these particulars which are merely given for the purpose of information.

20-09-2023

| Patent document cited in search report | | Publication date | Patent family member(s) | | Publication date |
|---|---|---|---|---|---|
| US 2016153904 | A1 | 02-06-2016 | JP | 6018711 B2 | 02-11-2016 |
| | | | JP | WO2015033394 A1 | 02-03-2017 |
| | | | US | 2016153904 A1 | 02-06-2016 |
| | | | WO | 2015033394 A1 | 12-03-2015 |
| WO 2022162224 | A1 | 04-08-2022 | NONE | | |
| US 2017059299 | A1 | 02-03-2017 | US | 2017059299 A1 | 02-03-2017 |
| | | | WO | 2015121853 A1 | 20-08-2015 |

For more details about this annex : see Official Journal of the European Patent Office, No. 12/82